# EUROPEAN PATENT APPLICATION

(11) **EP 2 138 988 A1**
(43) Date of publication of application: **30.12.2009**
(21) Application number: 08158929.3
(22) Date of filing: 25.06.2008
(51) Int. Cl.: G08G 1/0962, B60K 28/02

(54) **Method for determining a driving demand value**

(71) Applicant: Ford Global Technologies, LLC, Dearborn, MI 48126 (US)
(72) Inventor: Markkula, Gustav, 41663 Göteborg (SE); Broström, Robert, 41758 Göteborg (SE); Engström, Johan, 41649 Göteborg (SE)
(74) Representative: Valea AB

(57) **Abstract**

The present invention relates to a method for determining a driving demand value (1:1) to be used for evaluating the work load experienced by a driver of a vehicle and caused by factors external of the vehicle, comprising
- providing output from a plurality of sensors (3:1-3:8) for detecting demanding events external of the vehicle resulting in a high work load experienced by the driver,
- applying at least one individual driving demand evaluation rule (2:1 - 2:10) to each individual output of each sensor (3:1 - 3:8) to evaluate whether a demanding event is detected or not,
- each individual driving demand evaluation rule (2:1 - 2:10) generating a binary detection response value which is set active if a demanding event is detected, and
- setting a high driving demand value (1:1) if at least one binary detection response value is active.

## Description

### TECHNICAL FIELD

The present invention relates to a method for determining a driving demand value to be used for evaluating the work load experienced by a driver of a vehicle and caused by factors external of the vehicle

### BACKGROUND OF THE INVENTION

Modern vehicles are equipped with an increasing number of in-vehicle information systems. Such information systems present information from a wide range of vehicle applications, from alert or warning applications intended to draw the driver's attention to an occurrence being vital for ensuring a safe ride (such as a forward collision warning application, an unfastened seat belt warning application, or an engine overheating warning application) to information or entertainment applications (such as a navigation application, a mobile telephone application or a radio/music player application). For the information presentation, each of the various applications will utilize some information presentation device, which may comprise various lamps, audible signals, displays etc. Some applications may share the same information presentation device, e. g. the telephone and the music player may utilize the same display to present information.

Nevertheless, the increasing number of in-vehicle applications leads to an increased amount of information that may be presented to a driver during the ride of the vehicle. In driving conditions that are relatively easily mastered, such as driving in close to constant speed on a straight road segment with fairly low traffic, the driver may be presumed to be able to perceive and process information from several information systems without risk of substantial impairment of his/her ability to safely control the vehicle. However, in more demanding driving conditions, for example at a crossroad with several surrounding vehicles, it may be suitable to restrict the amount of information presented from the in-vehicle applications, so as to ensure that the driver is not distracted from his/her most important task of safely controlling the vehicle.

To this end, numerous attempts have been made towards determining when the driving conditions are demanding, meaning that the amount of non-vital information presented to the driver should be restricted, and when the driving conditions are not demanding, meaning that the amount of information to the driver need not be restricted. Generally, many previous systems determine a workload value, which is intended to provide an estimation of the workload experienced by the driver in various situations. Using the workload value, alone or in combination with other parameters, it is determined whether any information to the driver should be suppressed or allowed.

In order to determine a workload value, information regarding the present driving situation must be gathered. To this end, the vehicle may be provided with a number of sensors which provide information regarding different aspects of the driving situation. In prior art systems, the different outputs from the sensors are combined using some elaborated compilation rule so as to provide the workload value. The compilation rule may be set using theoretical assumptions of the driver's behaviour and/or relations that have been obtained empirically.

US 2004/0113799 describes a method for estimating the workload placed on the driver of a vehicle. The method uses vehicle data, environment data and current task data to estimate the workload placed on the driver. Vehicle data is received from sensors located inside the vehicle, environment data may include external information such as outside air temperature, GPS data, and digital maps. Current task data includes data such as radio information and phone information (e.g. is the phone active). The workload estimate calculation includes specific driving conditions that are detected from information contained in the workload estimation data. The impact values of the driving conditions are calibrated, e.g. using a weighting scheme and then combined together. The combined value may then be normalised and output as a driving workload estimate. The combine function used to generate the driving workload estimate may include an additive, multiplicative or other type of combination. The value from the combine function may be normalised so as to produce a workload estimate within a pre-selected range e.g. 1-5, 1-10, 1-100. Thereafter, any number of threshold or threshold values may be applied to the workload estimate in order to control the amount of information to be presented to the driver.

EP 1 512 374 describes a method for estimating a workload for a driver of a vehicle comprising the following method steps - identifying a demanding event from a signal indicative of a task requiring driver attention - generating a high workload output signal under said demanding event, -generating a workload delay time frame which extends a period in time from a demanding event end point in time until a workload delay end point in time, and - maintaining a high workload output signal under said workload delay time frame. For determining whether a demanding event is occurring or not, a number of parameters may be determined by applying classifying conditions to incoming signals.

Generally, the number of sensors to provide input to the workload estimation system is increasing with increased demand for sophisticated workload estimations. Hence, the complexity of the workload determination systems is also increasing. A problem associated with the increasing complexity may be that a fault in the system, such as a malfunctioning sensor, may affect the final workload estimation value in a manner which is difficult to predict. A fault in the system might also lead to the workload estimation function not working at all until the faulty component is replaced. Hence, a complex workload estimation system using a large number of input components will also be subject to a large number of potential error sources. Another potential problem with increasing complexity of the workload estimation system is the increasing diversity of the sensors used. The evaluation of driving conditions based on the output from the various sensors, as well as the interpretation of the workload value, tend to require elaborate evaluation and/or compilation rules. Hence, redesigning the work load estimation system by e.g. the addition or removal of a sensor type or system, or the addition or removal of an application using the in-vehicle information system might require considerable work involving adaptations of the evaluation and/or compilation rules.

In view of the above, there is a need for a system for determining a workload value which is robust to faults occurring in the system.

There is also a need for a system for determining a workload value which is compatible with a wide variety of types of sensors.

Further, there is a need for a system for determining a workload value which is easily scalable.

Also, there is a need for a system for determining a workload value to which new types of applications and sensors may be easily added or removed.

In addition, there is a need for a system for determining a workload value which provides an output value which may easily be interpreted.

The object of the invention is to provide a system for determining a workload value which meets at least one of the above-mentioned needs.

### SUMMARY OF THE INVENTION

This object is achieved in accordance with the invention by a method for determining driving demand value to be used for evaluating the work load experienced by a driver of a vehicle and caused by factors external of the vehicle, comprising
- providing output from a plurality of sensors for detecting demanding events external of the vehicle resulting in a high work load experienced by the driver,
- applying driving demand evaluation rules to the individual outputs of each sensor, wherein each individual driving demand evaluation rule is associated with one single individual output, to evaluate whether a demanding event is detected or not,
- each individual driving demand evaluation rule generating a binary detection response value which is set active if a demanding event is detected, and
- setting a high driving demand value if at least one binary detection response value is active.

Advantageously, each individual driving demand evaluation rule is adapted such that each binary detection response value is set inactive if the corresponding sensor is faulty.

Preferably, the driving demand value is generated by applying the OR operator to the plurality of binary detection response values.

Advantageously, the sensors and the corresponding driving demand evaluation rules include at least one sensor and rule using the present motion state of the vehicle for detection of demanding events. The motion state may include the present forward and/or backward acceleration, speed or yaw rate of the vehicle. The sensors may include at least one of an accelerometer, a speed sensor, a gyro, and a steering wheel angle sensor.

Advantageously, the sensors and the corresponding driving demand evaluation rules include at least one sensor and rule using the surrounding environmental conditions of the vehicle for detection of demanding events. The environmental conditions may include at least one of the temperature, geographical location, surrounding stationary objects or surrounding moving objects. The sensors may include at least one of a thermometer, a GPS sensor, and a radar.

Advantageously, the sensors and the corresponding driving demand evaluation rules include at least one sensor and rule using the driver's behaviour for detecting demanding events. The sensor for evaluating the driver's behaviour may include at least one of eye-lid tracker, a head tracker, an eye tracker.

Advantageously, the sensors and the corresponding driving demand evaluation rules include at least one sensor and rule using the state of driver controlled vehicle applications with external function for detecting demanding events. The state of driver controlled vehicle applications with external function may include at least one of the turn indicator, the windshield wipers, the reverse gear.

Advantageously, the sensors and the corresponding driving demand evaluation rules include at least one sensor and rule using the vehicle's present geographical position for detecting demanding events. The vehicle's present geographical position may be determined using at least one GPS sensor. The driving demand evaluation rule may include comparing the vehicle's present geographical position with a map database, wherein the map database includes defined geographical demanding areas, and the detection response value is set active if the vehicle is positioned in a demanding area.

Advantageously, the geographical demanding areas in the map database may include situation areas where situations requiring a high workload of the driver are likely to appear, and waiting areas where the driver is waiting before a demanding situation. Preferably, a driving demand evaluation rule may include setting an active detection response value if the vehicle is approaching a situation area, and is deemed to reach said situation area within a predetermined threshold time.

The object of the invention is also achieved by a system for performing the method as described above.

The method may advantageously be used for allowing or denying a request for interaction with the driver of a vehicle from an in-vehicle application.

### BRIEF DESCRIPTION OF THE DRAWINGS

Fig. 1 is a block diagram illustrating an embodiment of a system and method for determining a driving demand value.
Figs 2a to 2e are illustrations of road segments for exemplifying a method for determining a property of a driver-vehicle-environment state including setting a detection response value indicative of the presence of an event being demanding for a driver of a vehicle, comprising: determining the vehicle's present geographic position and comparing the position with a map database, wherein the database includes pre-defined geographical demanding areas.
Fig. 3 is a block diagram illustrating an embodiment of a method and system where a driver-vehicle-environment state vector including different properties of a driver-vehicle-environment state are obtained. The first of the properties in the vector is in this case a driving demand value obtained by the method and system as illustrated in Fig. 1.
Fig. 4 is a flow chart illustrating an embodiment of a method and system for allowing or suppressing a request for presenting information to a user from an application in an in-vehicle information system.
Fig. 5 is an embodiment of a truth table for setting an action priority parameter of an action request.
Fig. 6 is an embodiment of a truth table for setting a driver-vehicle-environment state priority category for a driver-vehicle-environment state (DVE) vector.
Fig. 7 is an embodiment of a truth table including ongoing actions and requested actions as classified into different action property categories, which indicates whether the requested action is allowed to run in parallel with the ongoing actions or not.
Fig. 8 is an embodiment of a truth table of action priority parameters of ongoing actions and requested actions, the action priority parameters including a time criticality parameter and a safety criticality parameter, and the table indicating which requested actions may interrupt which ongoing actions.

### DETAILED DESCRIPTION OF PREFERRED EMBODIMENTS

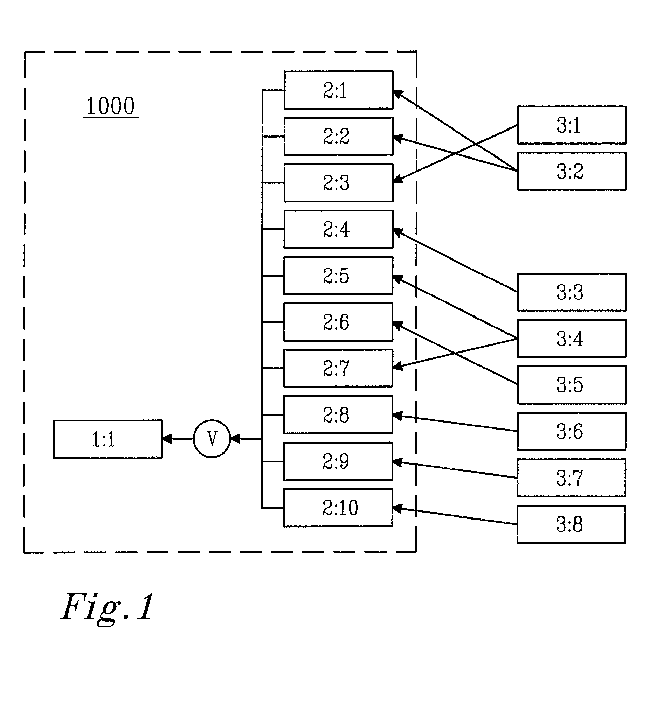

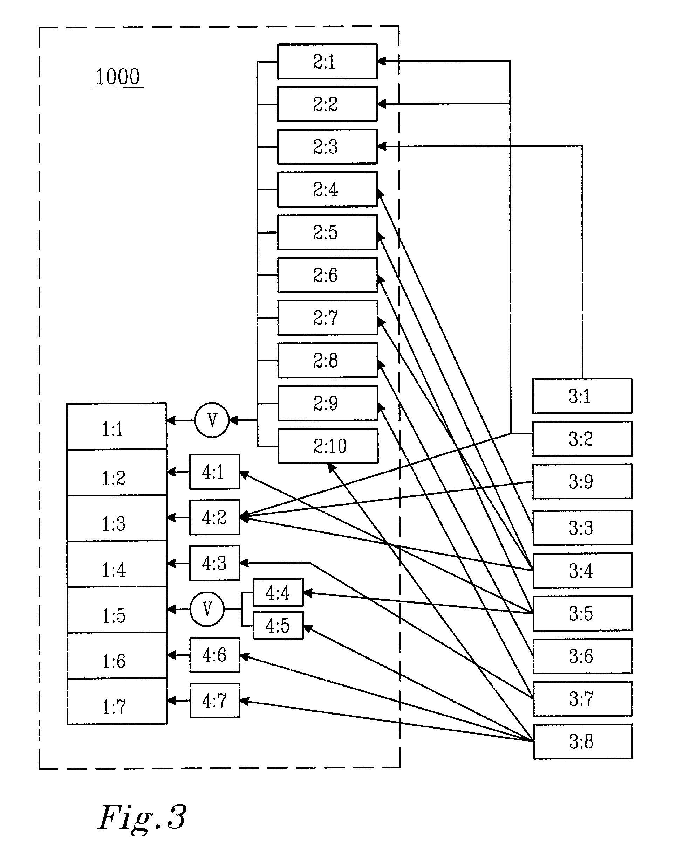

In Figs 1 and 3 reference numeral 1000 generally denote a processing device according to the present invention. The processing device comprises at least one processor, at least one memory, and at least one interface to at least one sensor. The interface may also comprise communicational capabilities and obtain sensor signals using an appropriate communication protocol such as using the CAN bus, Ethernet, I2C. Furthermore, the processor may comprise any suitable unit arranged to analyze the current "work environment", e.g. a microprocessor, a digital signal processor (DSP), an FPGA (field programmable gate array), or an ASIC (application specific integrated circuit). The memory may comprise a volatile memory and or a non-volatile memory, e.g. RAM, ROM, EEPROM, Flash, hard disk or similar memory types as understood by the skilled person.

The processing device is arranged to obtain one or several sensor signals relevant to the "work environment", pre condition the signal(s) and operate on them using a number of different analysis functions. The analysis may be implemented in either software or hardware instructions, but is advantageously implemented in software instruction sets stored in the memory and operated in the processing unit.

The different analysis functions will be discussed in more detail below.

Fig. 1 illustrates schematically an embodiment of a system and method for determining a driving demand value 1:1 to be used for evaluating the work load experienced by a driver of a vehicle and caused by factors external of the vehicle.

With "factors external of the vehicle" is meant external factors which must be estimated since no exact information regarding them is available internally in the vehicle. Generally, such factors are related to the driving task and include e.g. the surrounding traffic situation. However, external factors may be estimated using sensors which per se are positioned in the vehicle, such as head movement sensors used to track the head movements of the driver.

In contrast, information regarding factors internal in the vehicle are directly available in the vehicle, such as information whether the stereo or the telephone is active. Internal factors may also impact the total workload experienced by the driver. This will be more thoroughly explained below.

A plurality of sensors for detecting external demanding events resulting in a high workload experienced by the driver are arranged to provide output 3:1 - 3:8 indicative of said demanding events. Driving demand evaluation rules (2:1 - 2:10) are applied to the individual outputs of each sensor (3:1 - 3:8) to evaluate whether a demanding event is detected by the sensor or not. Each individual driving demand evaluation rule 2:1 - 2:10 is associated with one single individual output.

Each individual driving demand evaluation rule 2:1-2:10 generates a binary detection response value which is set active if a demanding event is detected. A high driving demand value (1:1) is set if at least one binary detection response value is set active.

It is understood that each individual driving demand evaluation rule being associated with one single individual output means that each individual driving demand evaluation rule is connected to only one individual output. Each output is connected to at least one, but possibly several, driving demand evaluation rules. Moreover, it is understood that one single sensor may provide several individual outputs.

This method is advantageous in that an individual driving demand evaluation rule is applied to each individual output of each sensor. Hence, each sensor is associated with at least one driving demand evaluation rule, making the system transparent to design, upgrade and repair. The driving demand evaluation rules generate binary detection response values, meaning that the different outputs from a diversity of sensors are transformed to binary response values being on the same level - true or false. This provides for an easy interpretation of the response values as no weighting factors or other interpretation methods need to be used to compare the response values with each other. Further, the method provides for an extremely simple compilation of the response values to a driving demand value, where the driving demand value is set high if at least one demanding event is detected, i. e. if at least one detecting response value is set high.

It is understood that with this method, addition, removal or replacement of a sensor is very convenient. To add a new sensor, the sensor and an associated driving demand evaluation rule providing a binary detection response value is applied. The compilation of the driving demand value will only be affected in that one more binary detection response value will be considered when determining if a demanding event is detected. The driving demand value per se will not be affected, that is, the resulting driving demand value will still be set either high or low. Consequently, the subsequent evaluation of the driving demand value will not be affected. Hence, the method is easily scalable. It is understood that the addition, removal or replacement of sensors may be made using a wide variety of sensors providing an equally wide diversity of outputs.

Further, the method is robust for faulty sensors. If a sensor is malfunctioning, the most common effect is that it simply ceases to function and hence it does not give any output at all. No output will normally result in no detection of a demanding situation and hence, as the driving demand evaluation rules generates binary detection response values, the corresponding binary detection response value is set low. As the driving demand value is binary and set high if at least one binary detection response value is high, it is understood that the only effect of this type of fault to the resulting driving demand value, is that the the type of demanding events detected by the rule or rules using the faulty sensor will not be detected in the evaluation. The evaluation of the remaining sensors and rules will be unaffected by the faulty sensor, which means that the effect of a faulty sensor to the entire driving demand evaluation process is minimised.

Advantageously, each driving demand evaluation rule is adapted such that each binary detection response value is set inactive if the corresponding sensor is faulty. Many sensor faults will simply result in no signal being obtained from the sensor, in which case no particular measures must be made in order to adapt the driving demand evaluation rule, as mentioned above. In other cases the sensor itself will report that is not functioning or that the data transmitted from the sensor is temporarily unreliable. However, if desired, the driving demand evaluation rule for each sensor may be adapted such that an evidently unrealistic signal from the sensor is interpreted as the sensor being faulty, in which case the binary detection response value is set inactive.

In the illustrated embodiment, the driving demand value is generated by applying the OR (V) operator to the plurality of binary detection response values. This is a straightforward way of determining whether at least one demanding event is detected or not, i.e. if one of the binary detection response values is set active or not. However, there are of course other logical operands that may be used to arrive at the same result, such as adding the binary detection response values together and setting the driving demand value high if the sum of binary detection response values set active is greater than zero (or possibly another value used as the "low" binary value). Such logical operands will be equivalent to applying the OR operator, although more cumbersomely formulated.

Advantageously, the sensors and the corresponding driving demand evaluation rules may include at least one sensor and rule using the present motion state of the vehicle for detection of demanding events. With motion state is meant the vehicle's motion in relation to the surroundings such as the forward and/or backward acceleration, speed and yaw rate. Examples of sensors suitable for detecting the state of movement are an accelerometer, a speed sensor, a gyro or a steering wheel angle sensor.

In the illustrated embodiment, the system uses the output from an accelerometer 3.1, a speed sensor 3.2, a gyro 3.3, and a steering wheel angle sensor 3.4 to determine the present state of motion of the vehicle. The respective outputs 3:1, 3:2, 3:3, 3:4 of each of these sensors are related to individual driving demand evaluation rules 2:1, 2:2, 2:3, 2:4 for generating binary detection response values.

The output 3:1 from the accelerometer is used by a driving demand evaluation rule 2:3 being a Lateral acceleration rule (LatAccRule). This rule is adapted such that the detection response value is set active when the absolute value of the lateral acceleration exceeds a predetermined threshold value.

The output 3:2 from the speed sensor is used by two different driving demand evaluation rules, 2:1 and 2:2.

The first driving demand evaluation rule using the speed sensor is a Positive longitudinal acceleration rule (PosLongAccRule) 2:1. This rule may acquire the longitudinal acceleration by numerical differentiation of a low pass filtered version of the vehicle's longitudinal speed signal obtained from the output 3:2 of the speed sensor. The driving demand evaluation rule sets the detection response value active when the longitudinal acceleration exceeds a predetermined threshold value.

The second driving demand evaluation rule using the speed sensor is a Negative longitudinal acceleration rule (NegLong Acc Rule) 2:2. This is the opposition of PosLongAccRule, wherein the detection response value is set active when the longitudinal acceleration is smaller than a negative threshold value.

The output 3:3 from the gyro is used by a driving demand evaluation rule being a Yaw rate rule 2:4 (YawRateRule). The detection response value is set active when the rate of yaw change (angular speed) of the vehicle exceeds a predetermined threshold value.

The output 3:4 from the steering wheel angle sensor is used by a driving demand evaluation rule being a Steering angle rule 2:5 (SteeringAngleRule). The detection response value is set active when the absolute value of the steering wheel angle exceeds a predetermined threshold value.

It is understood that the above-mentioned rules exemplify the principle of detecting demanding situations using information regarding the motion state of the vehicle. Generally, changes in the vehicle's state of motion, such as large accelerations or decelerations, lateral movements or sharp turns, are detected and used as indications of external situations occurring which are suspected to require high attention of the driver, thus subjecting the driver to a high workload.

Advantageously, the sensors and the corresponding driving demand evaluation rules may include at least one sensor and rule using the surrounding environmental conditions of the vehicle for detection of demanding events. With environmental conditions is meant the state of the surroundings, including the climate, surrounding stationary objects and surrounding moving objects. Hence, the environmental conditions may include at least one of the following: the outdoor temperature, the geographical location, the presence of surrounding stationary objects (such as obstacles) or moving objects (such as surrounding traffic). Sensors suitable for detecting environmental conditions may be thermometers, humidity sensors, radar, cameras, friction sensors and GPS sensors.

In the illustrated embodiment, the output from a GPS sensor 3:6 and the output of a radar 3:7 are used to provide information for detecting the surrounding environmental conditions.

The output 3:6 from a GPS sensor is used by a corresponding driving demand detection rule, being a Map position rule (MapPosition Rule) 2:8. Generally, this is a rule using the positioning data received from the vehicle's GPS sensor in combination with a map database to determine whether the vehicle is currently at a location that can be deemed demanding a *priori,* in which case the detection response value is set active. Generally, the driving demand evaluation rule includes comparing the vehicle's present geographical position with a map database, wherein the map database includes pre-defined geographical demanding areas, and the detection response value is set active if the vehicle is positioned in a demanding area.

The demanding areas could include road segments that are believed to be demanding such as crossings, roundabouts, lane crossings or the like, which are generally stationary environmental conditions. However, it is conceivable to use an updatable database which may include information that is not stationary such as ongoing road repairs.

There are numerous ways of constructing the rule and the map database so as to use of the geographical position of the vehicle. One such rule which have been found to be particularly advantageous will be described later on in this application.

Returning to the illustrated embodiment, the output 3:7 of the radar is used by a driving demand detection rule being a Time to collision rule (TTC rule) 2:9. A TTC rule would generally determine whether a driver is approaching a stationary or slower moving obstacle, such as another vehicle waiting at a traffic light, or a lead vehicle slowing down. This type of situation is generally deemed to be demanding. The TTC rule would, simply put, estimate the time to collision with the detected obstacle and set the driving demand value active if the time to collision is less than a pre-determined threshold value.

A useful TTC rule could be a similar rule as those conventionally used to issue forward collision warnings. For the purpose of setting a driving demand value, the threshold time limit for setting an active driving demand value should be higher than the threshold generally used for issuing a forward collision warning.

Advantageously, the sensors and the corresponding driving demand evaluation rules may include at least one sensor and rule using the driver's behaviour for detecting external demanding events. With the driver's behaviour is meant physical indications that an external demanding situation is at hand. The physical indications could include looking from side to side as before passing a crossing, repeated glancing over one shoulder before changing lane or the like. Suitable physical indications may be selected empirically from tests performed where drivers are subject to different driving situations. Hence, for evaluating the driver's behaviour, the input may include the driver's movement of the eyes, eye-lids or head. Sensors suitable for detecting the driver's behaviour are an eye or eye-lied tracker or a head tracker.

The corresponding driving demand detection rule or rules would be adapted so as to set a detection value active if the driver's behaviour indicates that high workload events are occurring. For example, numerous side looks may be used as an indication that the driver is changing lane which could be determined to be a high driving demand situation.

In the illustrated embodiment output 3:8 from a combined eye/eye-lid/head tracker sensor is used by a Head movement rule (HeadMovementRule) 2:10 for detecting demanding events.

Advantageously, the sensors and the corresponding driving demand evaluation rules may include at least one sensor and rule using the state of driver controlled vehicle applications with external function for detecting demanding events. With the state of driver controlled vehicle applications with external function is meant the setting on or off of external applications such as the turn indicators, the windshield wipers and the headlights.

In the illustrated embodiment, the states of different external functions are all available as in-vehicle CAN signals 3:5. Hence, the available state signals are used instead of sensors in this case.

A driving demand detection rule being a Turn indicator rule (TurnlndicatorRule) 2:7 uses the output relating to the turn indicator, and sets a detection response value active when the turn indicators are on to signal a turn.

Another driving demand detection rule being a Reverse rule (ReverseRule) 2:6 uses the output relating to the reverse gear and sets a detection response value active when the driver puts the vehicle in reverse gear.

All of the above-mentioned driving demand detection rules could suitably be combined with using a pre-defined minimum time during which detection must take place before the relevant detection response value is set active. In this case, the pre-defined minimum time may be adapted for each rule so as to balance the need for minimising the number of false detections while still having enough accuracy to detect the true demanding situations.

The driving demand detection rules could suitably be combined with a rule delay so as to avoid that the driving demand value rapidly changes in driving situations where one detection response value set high for a brief time period will be followed by another with a relatively short time gap in between, such as when braking and turning in a junction. The rule delay parameter may be set to maintain a detection response value high for some time (the delay) after the corresponding rule has detected a demanding embodiment. The rule delay is most suitably applied to driving demand detection rules that may set the detection response value high for brief time periods.

The application of rule delay can be determined using the demanding areas. For example, if the vehicle has left the centre of a situation area within a demanding area, and the situation area is a crossing, no rule delay will be applied to driving demand detection rules once the centre of the crossing is passed by the vehicle. The driving demand detection rules involved may be rules connected to e.g. turning, acceleration, and blinkers.

From the above description of an example system, it is understood that a wide range of sensors may be used, and that the options when designing a system are virtually endless. New types of sensors and/or advancements in the understanding of what properties may be used to indicate that the driver is experiencing a relatively high workload may alter the design of the workload estimation systems. Hence, the above-mentioned need for a method and system that may easily be adapted to new types of rules and sensors, to add sensors and to interchange sensors is more thoroughly understood.

As mentioned above, the sensors and the corresponding driving demand evaluation rules may include at least one sensor and rule using the surrounding environmental conditions of the vehicle for detection of demanding events. In particular, the environmental conditions may include the current geographical position of the vehicle, which is obtainable e.g. by a GPS sensor. In the illustrated embodiment, the output from a GPS sensor 3:6 and the output of a radar 3:7 are used to provide information for detecting the surrounding environmental conditions.

The driving demand evaluation rule may include comparing the vehicle's present geographical position with a map database, wherein the map database includes predefined geographical demanding areas, and the detection response value is set active if the vehicle is positioned in a demanding area.

In the following, a method for determining a property of a driver-vehicle-environment state including setting a detection response value indicative of the presence of an event being demanding for a driver of a vehicle will be described. This method may be used in a method for determining a driving demand value as described above, in which case the method will be used in one of the driving demand evaluation rules above. However, the method for determining a property of a driver-vehicle-environment state to be described can also be used with other systems and methods. In other words, the detection response value obtained by the method may be used for evaluating the workload of a driver using other estimation methods than setting a driving demand value active if at least one of a number of demand detection rules corresponding to sensors is set active. However, the combination of the two methods is believed to be particularly advantageous. One such combination is illustrated in the drawings.

Generally, the method for determining a property of a driver-vehicle-environment state includes setting a detection response value indicative of the presence of an event being demanding for a driver of a vehicle, comprises
- determining the vehicle's present geographic position;
- determining whether the vehicle's present geographic position is within a geographic demanding area, said demanding area being defined to include:
   -- a situation area where driving situations requiring a high workload of the driver are likely to appear, and
   -- at least one waiting area where the driver is expected to wait before the situation area; and ,
- setting the detection response value active if the vehicle is positioned in a demanding area.

It is believed that this method differs from previously proposed methods particularly in that the demanding areas include not only situation areas, which could be inside roundabouts, crossings, pedestrian crossings etc, but also waiting areas where the driver is waiting before a situation area. Waiting areas have previously not been perceived as demanding. In contrast, in some earlier systems, the fact that the vehicle is standing still when waiting has been used as an indication that the waiting situation is not demanding.

However, the inventors have realised that in the waiting situations the driver must often make decisions based on observations of the surrounding traffic, which may presently be inside the situation area. Further, traffic lights and road signs must be taken into account. In all, the preparation for entering into the situation area is demanding in itself, indicating a relatively high workload for the driver. This has not been captured by previous workload estimation systems.

In accordance with the method, the waiting areas are defined geographically, that is spatially. Hence, when waiting in a waiting area it is of no importance within which time period the vehicle will reach the situation area. Instead, the presence of the vehicle in a demanding area (being a waiting area or a situation area) is enough to generate an active detection response value. Accordingly, it is not relevant whether the vehicle is standing still or not when being within the situation area or the waiting area.

The determination whether the vehicle's present geographic position is within a pre-defined demanding area may be performed by applying a demanding area evaluation rule to the information regarding the geographic position obtained in the map database.

Such a rule may analyse database information regarding the surroundings of the vehicle to determine whether the vehicle is in a demanding area such as in or waiting before a crossing. The evaluation rule then includes the definitions of demanding areas to be applied to the database.

Alternatively, the determination whether the vehicle's present geographic position is within a pre-defined demanding area may be performed by retrieving information regarding pre-defined geographic demanding areas contained in the map database. In this method, the definitions of demanding areas have already been applied to the various traffic situations occurring in the database, resulting in a map database including information regarding demanding areas. This information may then be retrieved from the database.

The pre-defined demanding areas may advantageously include at least one of:
intersections, roundabouts, highway entrances, and sharp turns.

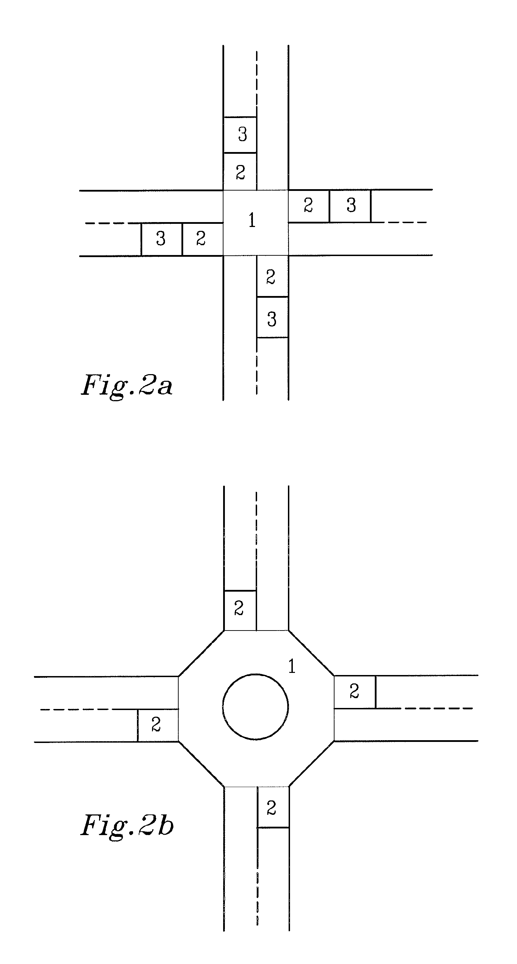

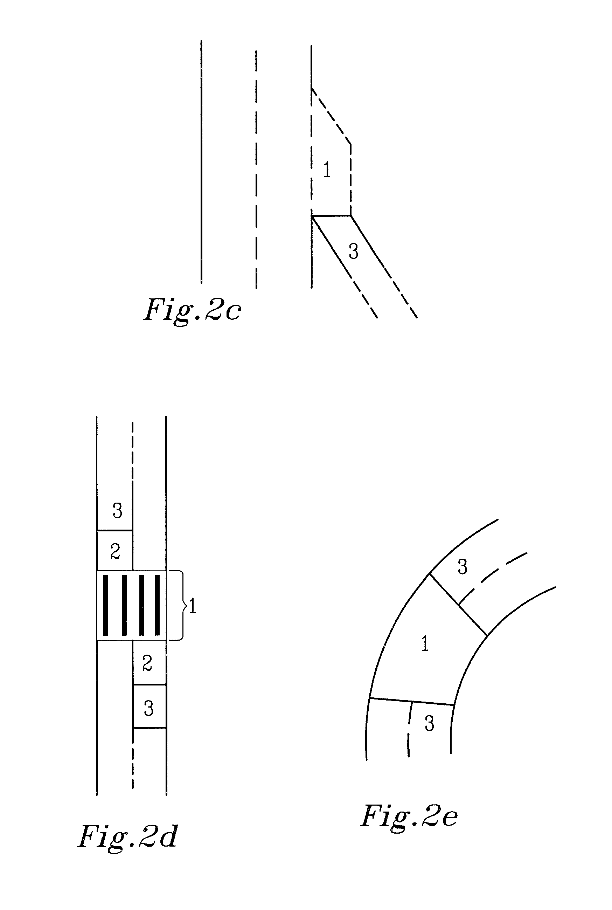

Figs 2a to 2e illustrate different examples of situation areas 1 and waiting areas 2. Fig. 2a illustrates a crossing where the crossing per se is a situation area 1, and waiting areas 2 are found at the four different entrances of the crossing. Fig. 2b illustrates a roundabout where similarly the roundabout per se is a situation area 1, and waiting areas 2 are found at the four different entrances to the roundabout. Fig. 2d illustrates a pedestrian crossing where the crossing per se is a situation area 1 and the road segments just before the crossing are waiting areas 2.

As is readily understood from the illustrated examples, the geographical demanding areas, which include both the situation areas and the waiting areas, may advantageously have an irregular shape. Having an irregular shape in this case means that the demanding area, if measured from the centre of the situation area, will have a greater extension towards entrance lanes to the situation area (where the waiting areas are found) than towards the exit lanes from the situation areas. This is in contrast to systems where a demanding area having a regular shape is defined by forming a circle with a constant radius around the centre of a situation area such as a crossroad. The irregular shape is advantageous in that it allows for including the waiting areas in the demanding areas while excluding the exit areas where the vehicle leaves the situation area. Hence, the resulting demanding areas are more accurately depicting the workload experienced by a driver than previous systems, as the demanding event signal may be set inactive as soon as the vehicle leaves the situation area.

The method may advantageously further comprise setting the detection response value active if the vehicle is approaching a demanding area and is deemed to reach within a predetermined time limit. An example of a suitable predetermined time limit could be about 4s.

The determination of if a vehicle is approaching a demanding area may be accomplished using information regarding the vehicle's current speed and acceleration. Hence, there will be no pre-defined geographical "approaching areas" in the map database or in the evaluation rule, as the size of the road segment in which the vehicle will be determined to approach a demanding area will depend on the vehicle's current speed. For illustration, road segments where the vehicle might be deemed to approach a demanding area are nevertheless denoted as portions 3 in the illustrations 2a-2e, though it should be understood that these road segments are not geographically defined as are the situation areas 1 or the waiting areas 2. 2a, 2b, and 2d illustrate situations where the vehicle will be deemed to approach the demanding areas in portions 3. In these cases, the approaching stage will come immediately before the geographical waiting areas 2. In Fig. 2c, which illustrates a lane crossing, there is no defined waiting area. Instead, the approaching portion 3 is found immediately before the situation area 1. This is the case also in the example of Fig. 2e, illustrating a sharp turn, where the turn per se is deemed to be a situation area 1, and approaching portions are found immediately next to it.

By combining the method including a map database above and the sensors and corresponding rules as previously exemplified an active driving demand value may be generated in a number of different driving situations that may be perceived as demanding, such as sharp turns, intersections or roundabouts, pedestrian/bike/tram/train crossing, hard braking, large acceleration, overtaking, changing lanes, changing lanes or merging in busy traffic, entering highway, driving in reverse gear , approaching a slower vehicle, waiting to turn or cross or looking for directions. Hence a highly versatile and useful system is obtained.

The geographical position of the vehicle may be determined as described above using a GPS-sensor. However, other methods for determination of geographic positions may also be used such as mobile phone triangulation methods or methods using information regarding the direction and speed of the vehicle to detect its position. The latter may be useful i. al. in situations where a GPS-signal is not available, such as in a tunnel.

The driving demand value to be used for evaluating the work load experienced by a driver of a vehicle and caused by factors external of the vehicle as determined for example by means of the method described above is one highly useful parameter for determining a property of a driver-vehicle-environment state.

However, other properties of a driver-vehicle-environment state are conceivable and may be used separately or in combination with the driving demand value. Such properties may be obtained at least partly using the same sensors that may be used for determining the driving demand value.

In the following, it will be described how different properties of a driver-vehicle-environment state capturing different aspects being relevant for evaluating the workload experienced by a driver may be obtained. In the illustrated exemplary embodiment, the properties are compiled in a driver-vehicle-environment state state vector, which in turn is useful for evaluation of the workload as will be described later on.

Fig. 3 illustrates a system where a driver-vehicle-environment state state vector 1:1 - 1:7 including different properties of a driver-vehicle-environment state are obtained. The different values in the vector relates to different aspects of the driver-vehicle-environment state. The first value 1:1 of the vector is a driving demand parameter. In the illustrated case, the driving demand parameter is obtained as explained in relation to Fig. 1. This is naturally believed to be particularly advantageous, but for the general purpose of forming a driver-vehicle-environment state state vector, a driving demand parameter could be obtained using some other evaluation method. Regardless of the evaluation method, the driving demand parameter may advantageously be binary, so as to be set active if a demanding situation is detected.

Advantageously, the property of driver-vehicle-environment state may include a driving mode parameter indicating whether the vehicle is in use or not.

In the illustrated embodiment, the second value in the vector is a driving mode value 1:2. This parameter is used to provide information whether the vehicle is in use or not. It may use basic CAN indicators 3:5 as input, and in this case, only the information whether the engine has been turned off or on for a certain duration or not. Hence, the parameter essentially tells whether the vehicle is parked or driving.

Advantageously, the method for determining a property of a present driver-vehicle-environment state further may include setting a driving complexity parameter indicating the complexity of the driving situation.

The third value in the vector is a driving complexity value 1:3. This parameter is intended to detect situations that are demanding on a larger time scale than the driving demand parameter 1:1. Whereas the driving demand parameter 1:1 is set active in a demanding local situation such as an intersection, the driving complexity value 1:3 is supposed to be constantly active throughout an entire sequence of intersections (e.g. in an urban environment) where the distance between the intersections is large enough for the momentary driving demand parameter to be inactive between them. This parameter can be used to block incoming information of low time criticality, such as an SMS or e-mail. Thus, the driving complexity value 1:3 uses a driving complexity computation 4:2 which returns an active value if the current driving environment is complex, and an inactive value if it is not. The driving complexity computation 4:2 may use information from the speed sensor 3:2, the steering wheel angle sensor 3:4, and a brake sensor 3:9 to determine the complexity of driving. Typically, an urban environment requires numerous braking with subsequent accelerations, and a relatively large number of turns.

Advantageously, the method for determining a property of a present driver-vehicle-environment state may include setting a vehicle following parameter indicating whether the driver is following another vehicle or not.

In the illustrated embodiment, the fourth value in the vector is a vehicle following parameter 1:4. This parameter is intended to tell whether or not the driver is currently following a lead vehicle. A vehicle following computation 4:2 uses information from a radar sensor 3:7 or the like, that is from a sensor which is capable of detecting objects surrounding the vehicle. The vehicle following computation 4:2 is to set the vehicle following parameter active if the driver follows a lead vehicle with a time headway that is less than a predetermined threshold value. The vehicle following parameter is set inactive if no lead vehicle is present, or if the lead vehicle is too far ahead.

Advantageously, the method for determining a property of a current driver-vehicle-environment state further includes setting a secondary task demand parameter, indicating whether the driver is presently occupied with a secondary task besides driving.

In the illustrated embodiment, the fifth value in the vector is a secondary task demand parameter 1:5. This parameter is intended to be set active if the driver is occupied with a secondary task, and to be set inactive if the driver is focusing on the driving task. The secondary task demand parameter is in the illustrated embodiment set active if either of two computation rules is active. The first computation rule is a button press detection 4:4. The button press detection 4:4 simply uses information from the basic CAN indicators of the vehicle 3:5 to determine whether the driver is currently manipulating infotainment buttons, and returns an active value if that is the case. Hence, the distraction caused at this stage is a distraction caused by factors inside of the vehicle. The second computation rule is a head movement computation 4:5 which uses information form an eye/head eyelid tracker 3:8 to determine whether the driver directs his attention to the interior of the vehicle, which is an indication that he is occupied with a secondary task, and results in an active value being set.

Advantageously, the method for determining a property of a current driver-vehicle-environment state may include setting a drowsiness parameter which is set active if the driver is evaluated to be drowsy or otherwise impaired.

In the illustrated embodiment, the sixth value in the vector is a drowsiness parameter 1:6, which is to be set active if it is determined that the driver is drowsy. There are numerous systems that can be used to set a drowsiness value. In the exemplified embodiment, the eye/head/eyelid tracker 3:8 is used in a drowsiness computation 4:6 to return the intended value.

Advantageously, the method for determining a property of a current driver-vehicle-environment state may include setting an eyes-off-road parameter which is set active if the driver is not directing his/her attention to the road.

In the illustrated embodiment, the seventh value in the vector is an eyes-off-road parameter 1:7, which is to be set active if the driver's visual focus is not on the road ahead of him. It uses an eyes off road computation 4:7 based on input from the eye/head/eyelid tracker 3:8.

As previously described, it is preferred that the property of the drive-vehicle environment forms a vector including all of the parameters described above. However, it is understood that parameters may be removed, added or replaced depending on the needs for a specific vehicle.

It is preferred that each of the parameters included in the property of the driver-vehicle-environment state returns a binary value which is set active or inactive depending on whether the corresponding situation is detected or not. Binary responses will facilitate the use of the property for evaluating the workload of a driver.

In particular, a property as obtained above is useful for estimating the workload of a driver with the purpose of determining whether or not to allow a request from an in-vehicle application system for display of information to the driver. In addition, the estimated workload could be used to determine in which manner information should be displayed to the driver.

It is understood that the above examples of parameters and sensors suitable in a driver-vehicle-environment state vector are illustrative examples only.

In the following, a method for allowing or suppressing a request for presenting information to a user from an application in an in-vehicle information system will be described. As will be described in relation to the illustrated embodiment the method may advantageously be combined with the methods and systems as described above. However, the method for allowing or suppressing a request may also be used separately, using other means to evaluate workload values than those described hereinbefore.

Generally, what is proposed is a method for allowing or suppressing a request for presenting information to a user from an application in an in-vehicle information system comprising:
- determining whether the request is allowable in view of a present workload value estimated from environmental sensor data, said workload value being representative of a workload experienced by the user; and
- evaluating whether the combined impressions perceived by the user from the ongoing applications presenting information to the user plus the requested information presentation are within the limits of a pre-determined maximum user impression capacity.

In accordance with the proposed method for allowing or suppressing a request for presenting information, a first step includes a determination based on an evaluation using environmental sensor data as input. In other words, this step is intended to take external factors into account. As the external factors surrounding the vehicle must necessarily be estimated, the knowledge of these must necessarily be incomplete. In the first step, the urgency of the request is hence evaluated in relation to an estimated workload experienced by a user and caused by external factors.

In a second step, the ongoing applications presenting information to the user are taken into account. As the ongoing applications are in-vehicle applications, complete and accurate information regarding these applications is available and easily accessible from the applications. In the second step, the urgency of the request is therefore evaluated in relation to the attention required by ongoing internal applications, and in view of a pre-determined maximum user impression capacity.

The pre-determined maximum user impression capacity decides how much information a user is deemed to be capable of handling in a certain situation. As some in-vehicle applications require more attention by the driver than others, the maximum user impression capacity should preferably take the different requirements of the applications into account.

By using separate steps for evaluating the allowability of a request in view of external factors or internal factors, it is believed that high accuracy in the evaluations is achieved. The detailed knowledge of internal factors is used in a separate step and is not mixed up with the step of evaluating external factors. As estimations will always be necessary for evaluating external factors, of which no exact information can be available. The external factors is instead, e.g. as described above, estimated based on information from different external sensors and evaluation rules used for interpreting the information gathered by the sensors.

Following the first two method steps, the method may further comprise determining whether the request is allowable in view of the availability of the input/output resources required by the requested application. Obviously, two applications cannot use the same output resource at the same time, which is why the input/output resources should be investigated before allowing a request.

Advantageously, the evaluation of whether the combined impressions are within the limits of a pre-determined maximum user impression capacity or not may be performed using a look-up table. A look-up table provides a simple means of indicating which applications may be run simultaneously.

To this end, the look-up table may include combinations of different information presentation activities and indications for each combination whether it is allowed or not in view of the maximum user impression capacity. Generally, which combinations to allow and which to deny may be determined empirically.

Further, the requests may be evaluated such that, if the request is deemed to be critical in view of safety, ongoing presentation activities are interrupted to allow the request.

Also, if the request is deemed to have to be executed within a certain time period, ongoing presentation activities may be interrupted to allow the request.

Further, if the request not deemed to be critical in view of safety or time, the request may be delayed.

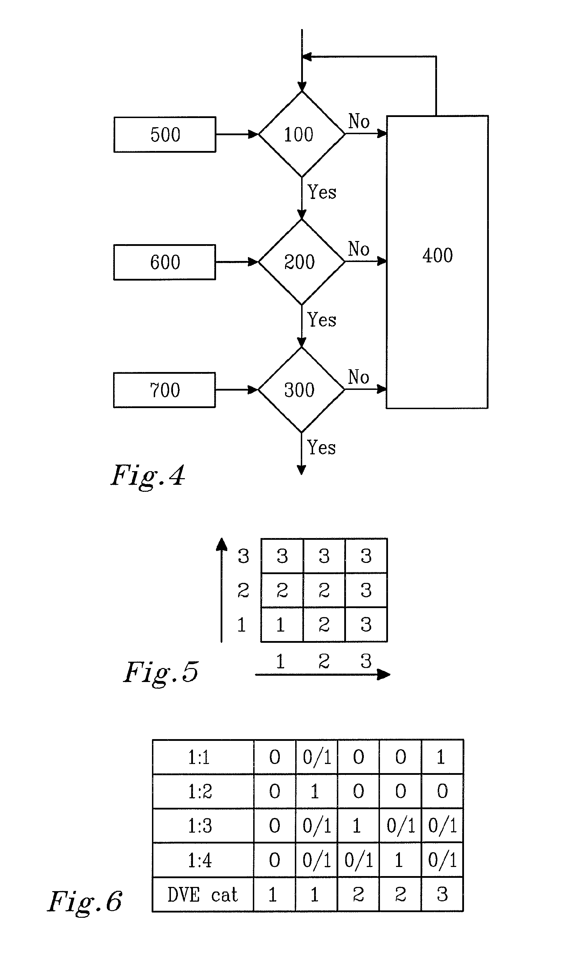

Fig. 4 illustrates schematically an embodiment of a method for allowing or suppressing a request for presenting information to a user from an application in an in-vehicle information system.

A request from an in-vehicle application to present information to the user first reaches a first method step 100, where it is determined whether the request is allowable in view of a present workload value 500 estimated from environmental sensor data, said workload value being representative of a workload experienced by the user.

The workload value 500 could be any type of workload value. However, it is believed to be particularly advantageous if using a driver-vehicle-environment state state vector 1:1 - 1:7 as described above and as illustrated in Fig. 3.

If the request is not allowed in view of the present workload value 500, the application is told to wait and the action request is stored in a waiting buffer 400. When any change of state is detected, e.g. the DVE state is altered or a function is finalised, the request in the waiting buffer 400 is re-evaluated. Alternatively, the request could be re-evaluated after a certain time interval.

If the request is allowed, it is passed on to the second method step 200, where it is determined whether the combined impressions perceived by the user from the ongoing applications presenting information to the user plus the requested information presentation are within the limits of a pre-determined maximum user impression capacity. The step uses input 600 regarding the ongoing applications.

If the request is not allowed in the second method step 200, it is stored in the waiting buffer 400. If the request is allowed, it is passed on to the final method step 300, where it is determined whether the required input/output resources are available using input 700 regarding the resources occupied by the ongoing activities. If not, the request is stored in the waiting buffer 400. If the resources are available, the request is allowed.
In order to determine the allowability of the requests, each request is provided with a safety criticality parameter and/or a time criticality parameter.

The time and safety criticality parameters may each be used to classify the urgency of the request in view of time or safety, into a suitable number of categories. A total action priority of the request may be obtained by evaluating the time and safety criticality parameters of different requests. If using e.g. three categories of safety criticality parameters and three categories of time criticality parameters (low, medium, high), to classify the action request, nine different action priority classes of requests will be obtained. This example is illustrated in Fig. 5, showing the action categories of the requests.

Apart from the action priority, the action request may include an action duration, being the time of duration of the action, if known. The horizontal axis indicates time criticality in three categories, and the vertical axis indicates safety criticality in three categories. The resulting action category is found in the table, also in three levels (low-1, medium - 2, high - 3).

Further, the action request may specify the input/output resources needed by the application in order to perform the action using one or several requested resource parameters.

Preferably, the action request may include an action property parameter, serving the purpose of categorising the action depending on properties being relevant for determining whether or not a certain action may occur simultaneously with other actions. This will be described in more detail below.

Advantageously, the action request may include a resource takeover type parameter, preferably being a binary parameter, indicating whether the requested input/output resources will be allocated permanently or temporarily.

For the determination of allowing or suppressing a request in the first step 100 of the method, the action category of the request must be evaluated in view of the present workload value estimated from environmental sensor data. If the workload value is presented in the form of a driver-vehicle-environment state vector as suggested above, it is advantageous to classify the different conceivable values of the vector into a vector state category.

An example of a truth table that may be used to determine the classification of a vector state category may be found in Fig. 6. In this embodiment, only the first four values of a DVE state vector such as described above is taken into account. The values correspond to different situations briefly as follows: 1:1 - driving demand is high; 1:2 - vehicle is parked; 1:3 high environment complexity; 1:4 - vehicle following situation. (See the more thorough explanation above.)

In the table, 1 denotes that a value is set active, and 0 that a value is set inactive. The DVE state category is set to 1, 2 or 3 (low, medium or high) depending on the different conditions of the DVE state parameter.

Assuming that a requested action has action category i, and the current DVE state category is j. Then the action may be presented if i ≥ j, otherwise not.

For example, if a driver is in a middle of a roundabout, the driving demand will be set active. Thus, according to the truth table the current DVE state belongs to DVE state category 3. In the middle of the roundabout someone calls on the mobile phone. Assuming that an incoming phone all has time criticality 2 and safety criticality 1, the request has the action category 2. Since 2<3, the phone call will be put on hold. If, instead of a phone call, a brake failure message had been issued, the action category of the brake failure message would be 3, and hence it would be allowed in the first method step 100.

If a request is allowed to pass to the second method step 200, it will be determined whether the requested action may go on simultaneously with the ongoing application or not. To determine this, a communication protocol may be set up, based on rules for simultaneous presentation.

To this end, the request may be provided with an action property for determining which combinations of different applications are allowable. The action property shall thus give information regarding the type of the request in view of presentation properties, or properties relating to the attention required by the user. One particular case which may advantageously form a specific action priority is user initiated applications. User initiated applications may generally be set to be granted without delay.

Examples of action properties are the following:
0 - Default - an action that does not possess any of the properties below.
1- Exclusive - an action that cannot be presented at the same time as any other action
2- Passive - an action that does not require active participation from the driver (e.g listening to the radio), thus allowing concurrent actions.
3- User initiated - an action that is initiated by the user. Such an action should always be granted without delay.

For example, the parking aid system may be exclusive since every other system should be quiet when the parking aid is active. Calling someone on the mobile phone is a user initiated action. Listening to the radio is considered a passive action, whereas entering a number on the phone is an active action.

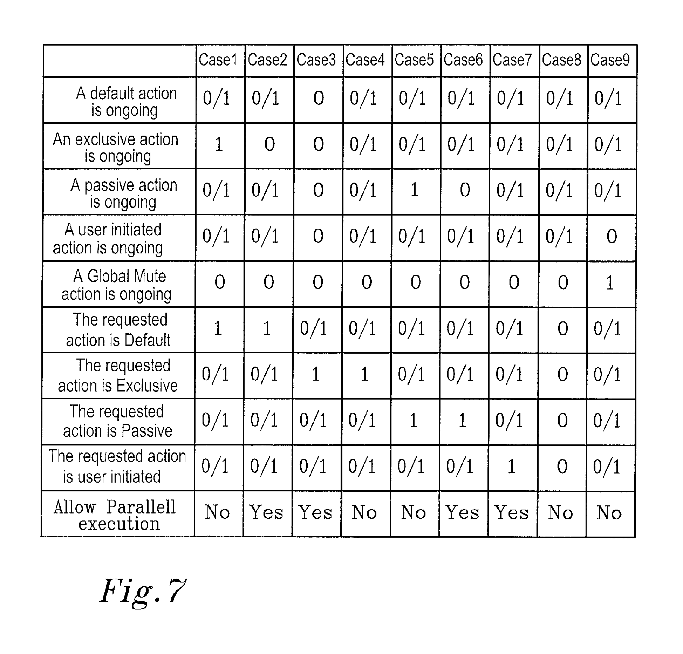

A truth table may be used to indicate which types of action that may be performed at the same time. The table lists different combinations of ongoing applications of the different action categories, and requested actions of different categories, and indicates whether the requested action shall be allowed to run in parallel with the ongoing applications. An example of such truth table is found in Fig. 7.

Hence, the table of Fig. 7 of the illustrated embodiment illustrates the evaluation of whether or not the combined impressions perceived by the user from the ongoing applications presenting information to the user plus the requested information presentation are within the limits of a pre-determined maximum user impression capacity. In other words, the pre-determined maximum user impression capacity is illustrated by the allowed or not-allowed combinations of the table.

Alternatively, a look-up table defining which combinations are allowable may be based on the specific events *per se.* That is, the request is not provided with action property parameters. Instead, each specific function (e.g. a phone call, route guidance) is listed in the look up table. This situation is advantageous if no unknown functions are involved.

Alternatively, a look-up table based on classifications such as action property parameters and specific functions may be used.

In all of the above-mentioned look-up table alternatives, the predetermined maximum user impression capacity is not a parameter to which some workload value or the like is compared. A table including different combinations has the advantage of being easy to overview and understand, which means that it is relatively simple to add, remove or displace different values or rules. The method enables use of binary values which makes the method reliable and predictable.

The parameters of the requested action and the ongoing actions are taken into account and used in the truth table so as to determine whether the request shall be approved or not at the second step 200 of the method.

As explained above, even if the request is allowed according to the second step 200, it may nevertheless be refused if the evaluation of the input/output resources made in the third step 300 finds that the necessary resources are not available.

If a request is not allowed to run in parallel with the ongoing actions, it is advantageous to determine whether the urgency of the request motivates interruption of an ongoing action in order to give room for the requested action.

For this determination, the time and/or safety criticality parameters as mentioned above may be used again. In this case, the time and safety criticality parameters may suitably be evaluated separately, which is why the combined action category as illustrated in the table of Fig. 5 is not used.

Again, it is understood that by using tables, the evaluation of the parameters may be performed as desired considering the specific occurring situations.

In such a table, 1 may denote that the ongoing action may be interrupted, and 0 that the ongoing action may not be interrupted. If the ongoing action may be interrupted, the interruption may be either permanent or temporary, depending on whether the new action is permanent or limited in time. If the ongoing action is interrupted temporarily the corresponding application is told to pause the interrupted action, and the interrupted action is placed in the waiting buffer.

If an action request passes the second step of the method, directly or on condition of interrupting an ongoing action, in this embodiment a third method step follows in which it is controlled whether the requested input/output resources are available. For each request, it is controlled whether the resources are available. If so, they are allocated to the request, and the request will finally be allowed or executed. If only not all of the requested resources are available, the application sending the request may decide whether the action shall be performed with limited resources or put in the waiting buffer.

For interrupting ongoing actions at this stage, a similar procedure may be performed as described above in relation to interruption at step two of the method. A lookup table may be used, which if desired could include different interruption rules than those of the second step.

It is understood that the action priority parameters may also be used in when the waiting buffer decides which of the requests in the waiting buffer that shall be resent to the beginning of the method. Also the other request parameters may be used to make suitable selections of which requests have a high possibility of being granted.

As regards the waiting buffer, existing waiting buffer systems may be used in the context of the methods and systems described herein. The same applies for the step of checking that the requested input/output resources are available for an action request.

The methods and systems may also be combined with other known methods and systems. For example, the decision whether to allow or not a request may take the duration of an action request into account, as described e.g. in EP 1 512 374.

The person skilled in the art will readily understand that numerous alternatives and embodiments are possible within the scope of the appended claims.

## Claims

1. Method for determining a driving demand value (1:1) to be used for evaluating the work load experienced by a driver of a vehicle and caused by factors external of the vehicle, comprising
- providing output from a plurality of sensors (3:1-3:8) for detecting demanding events external of the vehicle resulting in a high work load experienced by the driver,
- applying driving demand evaluation rules (2:1 - 2:10) to the individual outputs of each sensor (3:1 - 3:8), wherein each individual driving demand evaluation rule is associated with one single individual output, to evaluate whether a demanding event is detected or not,
- each individual driving demand evaluation rule (2:1 - 2:10) generating a binary detection response value which is set active if a demanding event is detected, and
- setting a high driving demand value (1:1) if at least one binary detection response value is active.

2. Method according to claim 1, wherein each individual driving demand evaluation rule (2:1-2:10) is adapted such that each binary detection response value is set inactive if the corresponding sensor (3:1 - 3:8) is faulty.

3. Method according to claim 1 or 2, wherein the driving demand value (1:1) is generated by applying an OR operator to the plurality of binary detection response values.

4. Method according to any one of the previous claims, wherein the sensors (3:1-3:8) and the corresponding driving demand evaluation rules (2:1-2:10) include at least one sensor and rule using the present motion state of the vehicle for detection of demanding events.

5. Method according to claim 4, wherein said motion state includes the present forward and/or backward acceleration, speed, or yaw rate of the vehicle.

6. Method according to claim 4 or 5, wherein said sensors (3:1-3:8) include at least one of an accelerometer, a speed sensor, a gyro, and a steering wheel angle sensor.

7. Method according to any one of the previous claims, wherein the sensors (3:1-3:8) and the corresponding driving demand evaluation rules (2:1 - 2:10) include at least one sensor and rule using the surrounding environmental conditions of the vehicle for detection of demanding events.

8. Method according to claim 7, wherein said environmental conditions include at least one of temperature, geographical location, surrounding stationary objects or surrounding moving objects.

9. Method according to claim 7 or 8, wherein said sensors (3:1-3:8) include at least one of a thermometer, a GPS sensor, and a radar.

10. Method according to any one of the previous claims, wherein the sensors (3:1-3:8) and the corresponding driving demand evaluation rules (2:1 - 2:10) include at least one sensor and rule using the driver's behaviour for detecting demanding events.

11. Method according to claim 10, wherein a sensor for evaluating the driver's behaviour includes at least one of eye-lid tracker, a head tracker, an eye tracker.

12. Method according to any one of the previous claims, wherein the sensors (3:1-3:8) and the corresponding driving demand evaluation rules (2:1 - 2:10) include at least one sensor and rule using the state of driver controlled vehicle applications with external function for detecting demanding events.

13. Method according to claim 12, wherein the state of driver controlled vehicle applications with external function includes at least one of turn indicator, windshield wipers, reverse gear.

14. Method according to any one of the previous claims, wherein the sensors (3:1-3:8) and the corresponding driving demand evaluation rules (2:1 - 2:10) include at least one sensor and rule using the vehicle's present geographical position for detecting demanding events.

15. Method according to claim 14, wherein the vehicle's present geographical position is determined using at least one GPS sensor.

16. Method according to claim 14 or 15, wherein the driving demand evaluation rule (2:1 - 2:10) includes comparing the vehicle's present geographical position with a map database, wherein the map database includes defined geographical demanding areas, and the detection response value is set active if the vehicle is positioned in a demanding area.

17. Method according to claim 16, wherein the geographical demanding areas in the map database include situation areas (1) where situations requiring a high workload of the driver are likely to appear, and waiting areas (2) where the driver is waiting before a situation area (1).

18. Method according to claim 17, wherein a driving demand evaluation rule includes setting an active detection response value if the vehicle is approaching a situation area (1), and is deemed to reach said situation area (1) within a predetermined threshold time.

19. System for determining a driving demand value to be used for evaluating a work load of a driver of a vehicle, comprising a processing device, communication means, and a plurality of sensors for providing information regarding the driving environment of the vehicle, wherein the processing device is arranged to receive sensor signals using the communication means, **characterised in that** each individual sensor (3:1-3:8) is associated with at least one individual sensor evaluation rule, wherein each of said rules provides a binary response value which is set high if the sensor evaluation rule concludes that a demanding event is present, and the driving demand value is set to indicate a high driving demand if at least one of said binary response values is set high.

20. System according to claim 19, wherein said system is adapted for performing the method of any one of the claims 1 to 18.

21. Use of a method in accordance with any one of the claims 1 to 18 for allowing or denying a request for interaction with the driver of a vehicle from an in-vehicle application.

22. A computer program stored in a computer readable storage medium, comprising instruction sets arranged to perform a method according to at least one of the claims 1 to 18 when the program is run on a processing device.
